# EUROPEAN PATENT APPLICATION

(11) **EP 4 616 781 A1**
(43) Date of publication of application: **17.09.2025**
(21) Application number: 24163568.9
(22) Date of filing: 14.03.2024
(51) Int. Cl.: A61B 1/00, A61B 1/04, A61B 1/06

(54) **METHOD AND SYSTEM FOR VISUALIZATION OF FAINT FLUORESCENCE IN SURGERY, SOFTWARE PROGRAM**

(71) Applicant: Quest Photonic Devices B.V., 1771 SR Wieringerwerf (NL)
(72) Inventor: TIEBIE, Kasper, 1761 AJ Anna Paulowna (NL); KOOPMAN, Thomas, 1013 WR Amsterdam (NL); HOVELING, Richelle Johanna Maria, 1623 JA Hoom (NL); BHOWMICK, Anubrata, 1782 JM Den Helder (NL)
(74) Representative: Seemann & Partner Patentanwälte mbB

(57) **Abstract**

The present disclosure relates to a method and a system (1) for visualization of faint fluorescence in surgery, as well as to a software program.

The method comprises the following steps:
- Emitting excitation light (12) from an excitation light source (6) onto an operation area (10) containing a faint fluorescence source (16), in particular parathyroid tissue exhibiting autofluorescence and/or fluorescent probes exhibiting fluorescence of similar intensity as the autofluorescence,
- Capturing one or more images of the operation area (10) at a wavelength range covering the wavelength range of the faint fluoresence source (16), which are called fluoresecent images, as well as one or more white light images,
- Performing image processing on the captured images by creating a virtual region of interest (20) in the white light image and/or the fluorescence image, wherein the fluorescence image is cut off outside the region of interest (20),
- Creating one or more false colour fluorescence images visible in the visible light spectrum from the one or more fluoresecent images,
- Creating one or more composite images (50) by overlaying the one or more false colour fluorescence images over the one or more white light images.

## Description

The present disclosure relates to a method and a system for the visualization of faint fluorescence in surgery, in particular open surgery, as well as a software program.

Fluorescence-guided surgery using fluorescent dyes is known in the art. Fluorescent dyes such as indocyanine green (ICG) or others are injected into tissue to be examined or operated on and, once the fluorescent dye has infiltrated the tissue, fluorescence excitation light is shone on the dye infused tissue, causing the fluorescent dye to emit fluorescence emission light, also called fluorescence light in short.

The basic principle of induced fluorescence is that the molecules making up fluorescent dyes have an excitation wavelength spectrum in which they are capable of capturing excitation light, absorbing the energy of the captured excitation light and transitioning to an excited state. The excited energy state of the fluorescent molecules is not stable. After a short period, excess energy is released from the molecule in the form of de-excitation light, also called fluorescence light leaving the fluorescent molecule once again in its basic energy state. The fluorescence light is slightly less energetic than the excitation light, which means that the fluorescent dyes' excitation spectrum is slightly shifted towards smaller wavelengths with respect to its fluorescence spectrum.

Therefore, excitation light used for exciting fluorescence in fluorescent dyes has a wavelength that is slightly offset towards smaller wavelengths than the fluorescence light that is to be observed. This is useful, since the amount of excitation light reflecting from the fluorescent dye infused tissue is orders of magnitude more intense than the resulting fluorescence light itself, and it would be impossible to detect fluorescence light in the presence of all the reflected excitation light. This slight shift in wavelength is exploited by using a wavelength filter configured to let light with the wavelength of the fluorescence light pass to shield off reflected excitation light from entering a camera.

Fluorescent dyes are not the only source of fluorescence in body tissues. Other sources include fluorescent probes used to label certain tissue types, such as cancerous tissues, and autofluorescent tissues such as the parathyroid. The parathyroid gland includes naturally occurring fluorescent molecules that emit fluorescence light in the near infrared spectrum when excited by excitation light of suitable wavelengths. However, compared with the above-described fluorescent dyes, the fluorescence signal emitted by autofluorescence is orders of magnitude weaker, i.e., orders of magnitude more prone to be outshone by excitation light or other sources such as ambient light having components in the spectral region of the autofluorescence.

The autofluorescence of the parathyroid is excited in a wavelength range around 785 nm and emits circa from 820 nm to 830 nm. This overlaps with, for instance, ICG, which emits in the range from 800 nm to 830 nm and is excited around 780 nm.

There are also autofluorescent colonic tissues and colonic cancers in the UV- and visible spectra, such as described for example in Li BH, Xie SS, "Autofluorescence excitation-emission matrices for diagnosis of colonic cancer", World J Gastroenterol. 2005 Jul 7;11(25):3931-4 or Bhaskar Banerjee M.D. et al., "Tryptophan autofluorescence imaging of neoplasms of the human colon," J. Biomed. Opt. 17(1) 016003 (1 February 2012).

The same holds for fluorescent probes used to label tissue types, such as cancerous tissues. Such probes and markers produce faint fluorescence signals of similar magnitude as the autofluorescence of the parathyroid gland, that is, orders of magnitude less than fluorescent dyes commonly used in fluorescence-guided surgery.

For these reasons, there is no fluorescence-guided surgery available that is based on faint fluorescence sources such as the autofluorescence of the parathyroid gland or fluorescent probes that image fluorescence light at similar faint intensities as the aforementioned autofluorescence.

It is an object of the present invention to provide means for utilizing faint fluorescence sources for fluorescence-guided surgery, in particular open surgery.

This object is achieved by a method for visualization of faint fluorescence in surgery, in particular open surgery, comprising the following steps:
- Emitting excitation light from an excitation light source onto an operation area containing a faint fluorescence source, in particular parathyroid tissue exhibiting autofluorescence and/or fluorescent probes exhibiting fluorescence of similar intensity as the autofluorescence, as well as white light from a white light source,
- Capturing one or more fluorescence images of the operation area at a wavelength range of fluorescence light emitted by the faint fluorescence source as well as one or more white light images,
- Performing image processing on the captured images by creating a virtual region of interest in the white light image and/or the fluorescence image, wherein the fluorescence image is cut off outside the region of interest,
- Creating one or more false colour fluorescence images visible in the visible light spectrum from the one or more fluoresecence images,
- Creating one or more composite images by overlaying the one or more false colour fluorescence images over the one or more white light images.

By creating a virtual region of interest and cutting off the fluorescence image outside of this region of interest, the fluorescence will only be visible inside the region of interest, while outside the region of interest the composited imagery consists only of the white light image. This makes it much easier for a surgeon to identify the faint fluorescence source, as fluorescence light from outside of the region of interest does not interfere with the fluorescence light from the faint fluorescence source. In particular, the intensity of the false colour fluorescence image is normalized according to the intensity distribution inside the region of interest. Normally, the fluorescence light from the faint fluorescence source may easily be outshone by excitation light or other sources such as ambient light having components in the same spectral region. By limiting the visibility of light in this spectral region to the region of interest, these sources are largely suppressed, resulting in a much larger contrast of the faint fluorescence source.

Furthermore, the brightness of fluorescence from the faint fluorescence source inside the region of interest may be used to adjust the gain of an imaging sensor, which might otherwise be set too low because of possible presence of specular reflections outside of the region of interest. Such bright spots outside the region of interest are ignored, thus focusing the control of sensor gain and/or image processing on the region of interest containing the sought-after faint fluorescence signal.

The images may be still images or videos. By using videos for both the white light images and the fluorescence images, a video feed is provided, in which the region of interest containing the parathyroid tissue or fluorescent probes is highlighted by providing fluorescence information on top of the white light video feed.

Preferably, the region of interest is created by using manual control elements in order to control the placement and/or shape of the region of interest. The manual control elements may be buttons, switches and/or a touchscreen display. With the help of these manual control elements, the surgeon may select the region of interest. For example, the surgeon may view a white light image of the operation area and select the region of interest based on this white light image. The surgeon may instead view a composite image consisting of an overlay of the false colour fluorescence image over the white light image and select the region of interest based on this composite imagery or the surgeon may select the region of interest solely based on the fluorescence image. The region of interest may have any shape and form, for example a circle, an ellipse, a rectangular shape or a shape conforming to the contours of an organ or tissue visible in the white light image.

The region of interest is preferably automatically repositioned after a movement, in order to realign the region of interest in relation to the faint fluorescence source in the one or more composite images. The movement may be a movement of the patient or a movement of an image capturing device used to capture the images. Such movements may occur accidently during surgeries, for example due to a slight shift of the patient or shaking of the image capturing device. However, such movements may change the position of the region of interest with respect to the faint fluorescence source. This is undesirable, as it decreases the visibility of the fluorescence in the composite image and may hinder the surgeon during the surgery. To offset this movement, the region of interest is automatically realigned to the faint fluorescence source. In this way, the movement does not impede the surgery.

According to another embodiment, the region of interest is automatically repositioned by an algorithm, which is trained to recognize a shape of an organ comprising or consisting of the faint fluorescence source, wherein the region of interest is realigned in relation to the organ. The algorithm may be an artificial neural network, which has been trained with white light image data of organs, for example parathyroid glands, in order to reliably detect these organs in white light images. Afterwards, the region of interest is realigned in relation to the organ in order to offset the movement.

According to another embodiment, the region of interest is automatically repositioned by performing image analysis on the white light image in order to identify an image region with a predominantly red wavelength spectrum, wherein the region of interest is realigned in relation to or set as the image region with a predominantly red wavelength spectrum. In surgery, the patient is typically covered by a blanket or cloth, with just a small opening around the area of operation. The incision made in open surgery is typically visible as a red region in white light image, in contrast to the surrounding skin and the blanket or cloth. This allows an identification of the incision by image analysis of the white light image, identifying the incision as the region with a predominantly red wavelength spectrum. Afterwards, the region of interest may be realigned such that it is centred on the region with a predominantly red wavelength spectrum or that it is set to equal the region with a predominantly red wavelength spectrum. This is an easy way to offset a movement during surgery and mask any light in the relevant wavelength spectrum from outside of the incision.

According to an embodiment, specular reflections are detected using high intensity information in the white light images, wherein areas comprising the specular reflections are deleted from the region of interest. These areas also reflect fluorescence light, in particular near infrared light, which causes high intensity spots to appear in the fluorescence image, which is unwanted. Thus, these regions are deleted from the region of interest, removing the fluorescence information in these areas from the composite image.

Preferably, the image processing step of the method further comprises increasing a contrast between the fluorescence light emitted by the faint fluorescence source and background.

Increasing the contrast between the fluorescence light emitted by the faint fluorescence source and the background helps in making the faint fluorescence signal visible over the background, which can then be masked out.

Preferably, the fluorescence images are captured using an image sensor at high gain settings and/or exposure. Due to the high gain settings and/or exposure, the sensor will go into saturation in parts of the image with medium to high light intensity, whereas the darker regions will be enhanced and occupy the dynamic range of the picture. Still, the faint fluorescence signal will occupy the lower part of the dynamic spectrum.

In embodiments, the contrast between the fluorescence light emitted by the faint fluorescence source and background is increased by applying an inverse gamma correction. A gamma correction is a nonlinear transformation of luminance values in video or still image systems. This nonlinear transformation darkens relatively dark areas in the image, whilst relatively light areas of the image keep their relatively light intensity.

However, in the present case, it is the dark parts of the fluorescence images that need to be contrast-enhanced, and it is therefore useful to use the inverse exponent of the logarithmic function, hence the name "inverse gamma correction". The inverse gamma correction may also be called decoding gamma or gamma expansion.

Depending on the setup and on the nature of the fluorescence light, the white light images and the fluorescence images are captured either simultaneously or alternately in embodiments. The simultaneous capturing of white light images and fluorescence images is practicable in case that the fluorescence signal lies outside of the visible light spectrum captured in the white light images and an image capturing device is available that comprises image sensors that are sensitive in the white light on the one hand and in the region of the fluorescence signal on the other hand.

If, on the other hand, the fluorescence light has a wavelength that falls within the white light spectrum, the whites light images and the fluorescence images cannot be captured simultaneously since the white light illumination would drown out the fluorescence signal. In such cases, it is possible to alternate between white light illumination and fluorescence illumination and to use pairs of a white light image and an immediately subsequently captured fluorescence light image, or vice versa, to provide the composite images.

It is also possible to interject a fluorescence image capturing once in a while in between otherwise uninterrupted sequences of white light images and to use the captured fluorescence image to be overlaid over each of the sequence of white light images, particularly if the sequence of white light images does not undergo rapid movements or such movements are identified and the location of the intermittent fluorescence image is adjusted to conform to the movement to be overlaid over the correct spot in each of the white light images. Such intermittent capturing of fluorescence images has the added benefit that there is less flicker in the illumination of the operating area. Movements can be offset by the methods described in relation to the realignment of the region of interest.

In embodiments, the image processing on the one or more fluorescence images comprises at least one of masking specular reflection, noise suppression and normalization. Specular reflections will show up as saturated regions of the images, either in the white light images, the fluorescence images or both. Such saturated regions can be excluded from further image processing in the fluorescence images and masked as background, for example by setting the brightness value of the afflicted pixels of the fluorescence image to zero, that is, black.

Noise suppression is a known technique in image processing and beneficial in the present context, since the fluorescence signals from faint fluorescence sources are very weak. Such weak signals have a low signal to noise ratio so that it might be difficult to sort out the faint fluorescence signal from the noise, depending on circumstances. Known noise suppression techniques are filtering over the adjacent pixels and taking mean values of the brightness of adjacent pixels or pixel clusters. Known removal algorithms reduce or remove the visibility of noise by smoothing the entire image, leaving areas near contrast boundaries. These methods can obscure fine, low contrast details, but in the present context, where the goal is to identify structures and tissues exhibiting faint fluorescence, this trade-off is acceptable in order to receive a clear signal identifying the structure or tissue in the first place.

The normalization performed during image processing of the one or more fluorescence images may be used in order to create the false colour images representing the fluorescence images. Normalization means that the dynamic range of a fluorescence image is focused on the interesting part of the fluorescence image, which in the case of faint fluorescence sources is the low intensity part of the fluorescence image. In embodiments, the normalization comprises a percentile normalization, in particular cutting off the darkest 1 to 2 % and the brightest 1 to 2 % of the pixels of the fluorescence image, which removes saturated areas as well as background areas without signal that only contain noise. Alternatively or in addition, the normalization can comprise selecting a bottom part of the brightness spectrum and spreading the brightness information contained therein over the full brightness spectrum, wherein in particular the bottom part of the brightness spectrum includes 0% to 20%, in particular 0% to 10%, in particular 0% to 5%, of the brightness spectrum of the input fluorescence images. The extent of the chosen region is to be adapted to the actual gain settings and signal distributions observed under the specific circumstances of the surgery.

The threshold below which the faint fluorescence signal is found and above which the signal should be discarded may also be adjusted automatically or manually by the surgeon or an assistance to the surgeon in order to gain optimal visibility of the faint fluorescence signal in the composite image. Automatic adjustment of the threshold may be achieved by automatic analysis of the signal amplitude distribution. For example, the normalization algorithm may start with a threshold of 30% of the maximum of the brightness of the raw fluorescence image and select an isolated spot in the fluorescence image with the highest intensity below that threshold and select a new threshold representing a brightness value just above that relatively brightest spot. By selecting an isolated spot it is avoided that the algorithm simply arrives at the border to a saturated region that is being masked out using the initial 30% threshold. If, however, there are other isolated spots that go into saturation using the lower threshold, the threshold is increased again until all isolated spots are shown without their centers going into saturation, i.e., the brightness in the center of the respective spot being greater than the threshold.

The object is further achieved by a system for the visualization of faint fluorescence in surgery, in particular open surgery, comprising
- a control device comprising an image processing unit,
- a light source device configured to produce excitation light and white light,
- an image capturing device configured to capture fluorescence images and white light images,
wherein the control device is configured to control the operation of the light source device and the image capturing device and to carry out the method steps of the method according to one of the previously described embodiments.

The image capturing device may be a camera. The image capturing device may comprise an image capture unit for the white light and a separate image capture unit for the fluorescence light. The control device may be a controller, in particular a computer. The control device is configured to control the light source device in order to emit the excitation light and the white light onto the operation area and to control the image capturing device in order to capture the one or more fluorescence images and the one or more white light images. The image processing unit performs the image processing, in particular the creation of the region of interest and/or the creation of the false colour fluorescence image and/or the creation of the composite imagery.

In particular, the light source device comprises an excitation light source and a white light source. The white light source may preferably a light source having no emission within the spectral range of the fluorescence signal of the faint fluorescence source, such as, for example, LEDs or laser sources. In particular, the system comprises a lighting and image capturing device, configured to emit the white light and the excitation light and to capture the white light image and the fluorescence image. Thus, the emission of the light and the capturing of the images is combined in a single device.

The object is additionally achieved by a software program configured to execute the method steps of the method according to one of the previously described embodiments when run on the control device of the system according to one of the previously described embodiments.

The system for the visualization of faint fluorescence in surgery and the software program embody the same advantages and characteristics as the method described above. Features mentioned with respect to the method are expressly applicable to the system and vice versa, as well as to the software program performing the control of the system components and the image processing part of the method, as well as receiving input from system components.

Further characteristics of the invention will become apparent from the description of the embodiments according to the invention together with the claims and the included drawings. Embodiments according to the invention can fulfil individual characteristics or a combination of several characteristics.

The invention is described below, without restricting the general intent of the invention, based on exemplary embodiments, wherein reference is made expressly to the drawings with regard to the disclosure of all details according to the invention that are not explained in greater detail in the text. The drawings show in:
- Fig. 1: a schematic simplified representation of a system for visualization of faint fluorescence in a surgery,
- Fig. 2: a schematic simplified representation of another system for visualization of faint fluorescence in an surgery,
- Fig. 3: a schematic simplified representation of a composite image of an operation area, consisting of an overlay of a white light image and a fluorescence image, and
- Fig. 4: a schematic simplified representation of method steps of a method for visualization of faint fluorescence in surgery

In the drawings, the same or similar types of elements or respectively corresponding parts are provided with the same reference numbers in order to prevent the item from needing to be reintroduced.

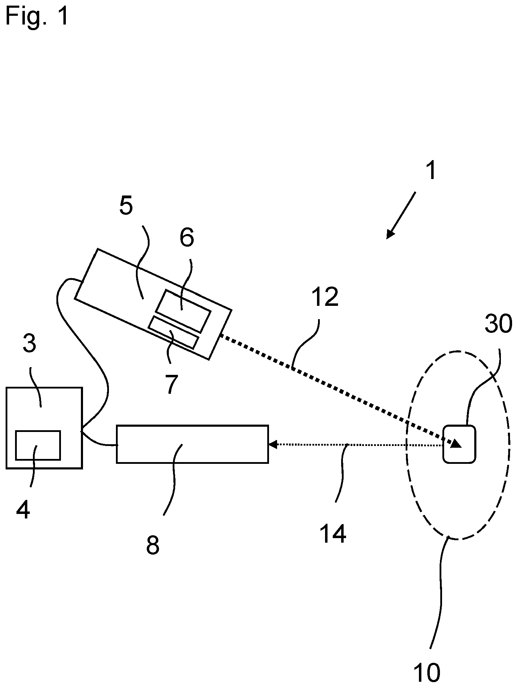

Figure 1 shows a schematic simplified representation of a system 1 for visualization of faint fluorescence in a surgery. The system 1 comprises a control device 3 with an image processing unit 4 that is implemented as either image processing software on a general purpose computer or as a dedicated image processor configured to process the imagery. The control device 3 is connected to and controls a light source device 5 and an image capturing device 8. The light source device 5 comprises both an excitation light source 6 and white light source 7 to illuminate an operation area 10. Excitation light 12 emitted by the light source device 5 stimulates autofluorescent tissue 30 in the operation area 10 or fluorescent probes exhibiting fluorescence of similar intensity as the autofluorescence. The autofluorescent tissue 30, for example parathyroid tissue, will emit fluorescence light 14 at a specific wavelength range. This wavelength range will typically be in the near infrared spectrum and will usually have no overlap with the white illumination light in order to separate the white light from the fluorescence light 14.

The image capturing device 8 is configured to capture imagery at this wavelengths range as well as white light imagery. Typically, the image capturing device 8 comprises an image capture unit for white light, for example a RGB unit, and an image capture unit for the fluorescence light 14. The imagery captured by the image capturing device 8 is transmitted to the image processing unit 4 of the control device 3. The image processing unit 4 performs image processing in order to enhance the visibility of specific features of interest in the operation area 10.

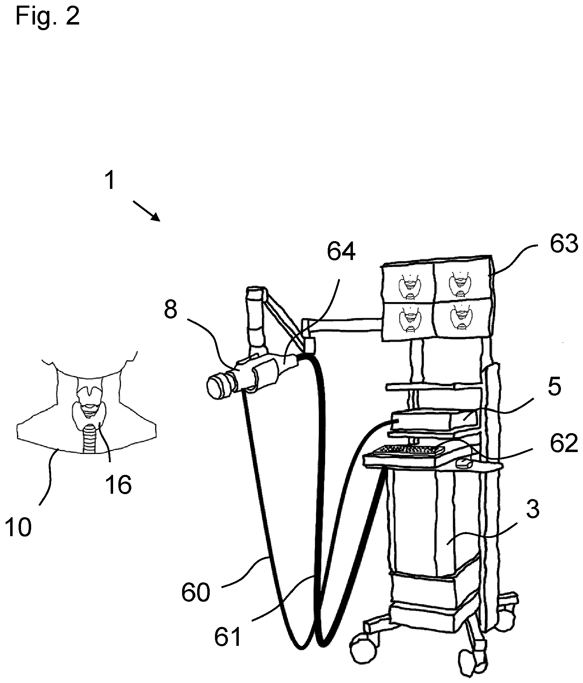

Figure 2 shows another embodiment of a system 1 for visualization of faint fluorescence in an open surgery. In this embodiment, the image capturing device 8 and the lighting are combined to a single lighting and image capturing device 64. The device 64 is connected via a fiber cable 60 to the light source device 5 in order to illuminate the operation area 10 of the parathyroid. The operation area 10 comprises a faint fluorescence source 16, e.g. the autofluorescent parathyroid gland. The image capturing device 8 captures a white light image and a fluorescence image of the operation area 10 and transmits the image information via data cable 61 to the control device 3, for example an industrial computer. The images may be displayed on the display 63. Manual control elements 62, for example a keyboard and a mouse, may be used by a surgeon to select a region of interest 20 in the images and/or to perform image processing. The image processing may comprise increasing a contrast between a signal from the faint fluorescence source 16 and a background, applying an inverse gamma correction, masking specular reflections, applying noise suppression and/or applying normalization.

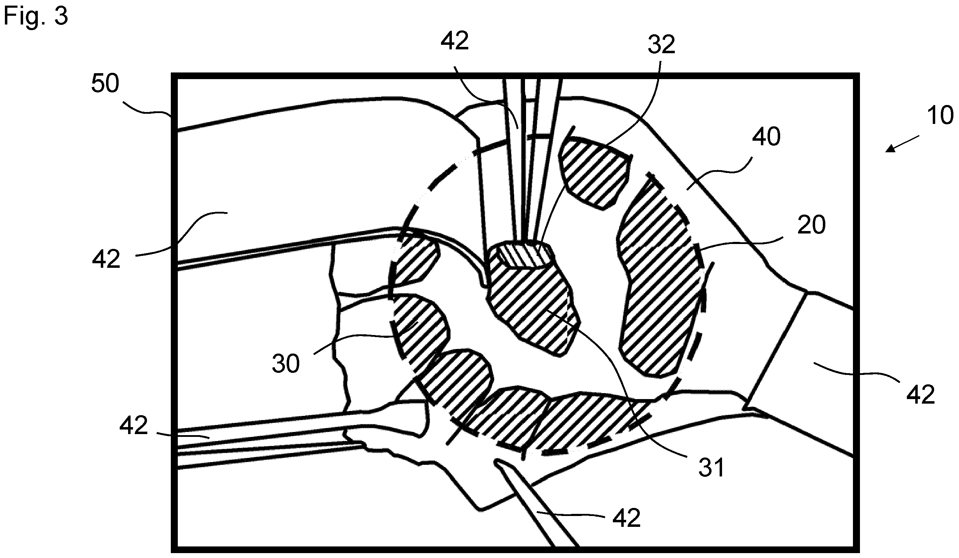

Figure 3 shows a schematic simplified representation of a composite image 50 created by the image processing unit 4. The composite image 50 may be displayed on display 63 and consists of an overlay of a false colour fluorescence image over a white light image. The operation area 10 shown in the composite image 50 comprises tissue 40 and several surgical tools 42 used during an open surgery. Fluorescence is represented by dashed lines in Figure 3. In the centre of the image, an autofluorescent organ 31, for example a parathyroid gland, is shown with a specific part emitting a particular fluorescent signal 32 of higher intensity then the fluorescence of the other autofluorescent tissue 30. Still, the intensity of the fluorescent signal 32 is much smaller than the intensity of the excitation light or other sources of light such as ambient light having components in the spectral region of the autofluorescence.

In order to enhance the contrast in the composite image 50, the surgeon may create or select a region of interest 20 in the image. The region of interest 20 is represented as a dashed circle in Figure 3, however it may have a different form and/or size. Outside of the region of interest 20, only the white light image is shown in the composite image 50, while the fluorescence image is cut off. The surgeon may select and place the region of interest 20 using the manual control elements 62 or select the region of interest 20 directly on the display 63 showing the composite image 50.

In case that the patient or the image capturing device 8 moves during the surgery, the region of interest 20 may no longer align with the organ 31. In order to offset such a movement, the system 1 is configured to track the movement and realign the region of interest 20 accordingly. In order to track the movement, the image processing unit 4 may perform shape detection on high intensity information in the white light image in order to detect specular reflection. Specular reflection will typically result in intensity spikes in the white light image, which are used to identity certain regions in the image and help correct the positioning of the region of interest 20. The specular reflections may also be detected by the image capture unit for the fluorescence light 14. In addition, the image processing unit 4 may utilize an algorithm trained to recognize the shape of the organ 31, e.g. the parathyroid, in order to correct the positioning of the region of interest 20. Also, the image processing unit 4 may position the region of interest 20 based on the colours in the white light image. Specifically, as tissue in an open surgery typically appears red in a white light image, the region of interest 20 is placed on a region predominantly in the red wavelength range.

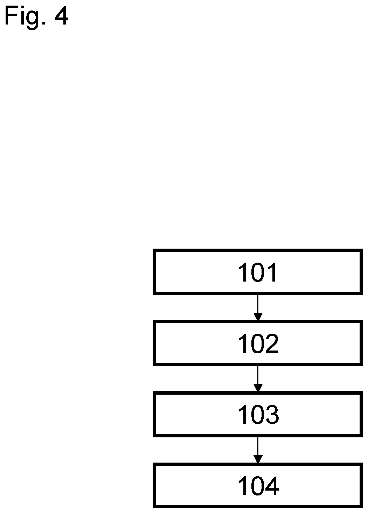

Figure 4 shows a schematic simplified representation of method steps of a method for visualization of faint fluorescence in surgery. At step 101, excitation light 12 is emitted from the excitation light source 6 onto the operation area 10 containing the faint fluorescence source, for example parathyroid tissue exhibiting autofluorescence. At step 102, one or more images of the operation area 10 at a wavelength range covering the wavelength range of the faint fluorescence source as well as one or more white light images are captured. At step 103, image processing is performed on the captured images. The image processing may include the creation of a virtual region of interest 20 in the white light image and/or the fluorescence image. Outside the region of interest 20, any image information of the fluorescence image is cut off. Image processing may also include increasing a contrast between the faint fluorescence source 16 and a background, applying an inverse gamma correction, masking specular reflections, applying noise suppression and/or applying normalization. At step 104, a false colour fluorescence image visible in the visible light spectrum is created from the fluorescent image. Then, at step 105, a composite image 50 is created by overlaying the false colour fluorescence image over the white light image. In this composite image 50, the false colour fluorescence is only visible inside the region of interest 20.

All named characteristics, including those taken from the drawings alone, and individual characteristics, which are disclosed in combination with other characteristics, are considered alone and in combination as important to the invention. Embodiments according to the invention can be fulfilled through individual characteristics or a combination of several characteristics. Features which are combined with the wording "in particular" or "especially" are to be treated as preferred embodiments.

### List of References

- 1: System
- 3: control device
- 4: image processing unit
- 5: light source device
- 6: excitation light source
- 7: white light source
- 8: image capturing device
- 10: operation area
- 12: excitation light
- 14: fluorescence light
- 16: faint fluorescence source
- 20: region of interest
- 30: autofluorescent tissue
- 31: organ
- 32: fluorescent signal
- 40: tissue
- 42: surgical tool
- 50: composite imagery
- 60: fiber cable
- 61: data cable
- 62: manual control elements
- 63: display
- 64: lighting and image capturing device
- 101 - 104: method step

## Claims

1. Method for visualization of faint fluorescence in surgery, in particular open surgery, comprising the following steps:
- emitting excitation light (12) from an excitation light source (6) onto an operation area (10) containing a faint fluorescence source (16), in particular parathyroid tissue exhibiting autofluorescence and/or fluorescent probes exhibiting fluorescence of similar intensity as the autofluorescence, as well as white light from a white light source (7),
- capturing one or more fluorescence images of the operation area (10) at a wavelength range of fluorescence light (14) emitted by the faint fluorescence source (16) as well as one or more white light images,
- performing image processing on the captured images by creating a virtual region of interest (20) in the white light image and/or the fluorescence image, wherein the fluorescence image is cut off outside the region of interest (20),
- creating one or more false colour fluorescence images visible in the visible light spectrum from the one or more fluoresecence images,
- creating one or more composite images (50) by overlaying the one or more false colour fluorescence images over the one or more white light images.

2. Method according to claim 1, wherein the region of interest (20) is created by using manual control elements (62) in order to control the placement and/or shape of the region of interest (20).

3. Method according to claim 1 or 2, wherein the region of interest (20) is automatically repositioned after a movement, in order to realign the region of interest (20) in relation to the faint fluoresecent source (16) in the one or more composite images (50).

4. Method according to claim 3, wherein the region of interest (20) is automatically repositioned by an algorithm, which is trained to recognize a shape of an organ (31) comprising or consisting of the faint fluorescence source (16), wherein the region of interest (20) is realigned in relation to the organ (31).

5. Method according to claim 3 or 4, wherein the region of interest (20) is automatically repositioned by performing image analysis on the white light image in order to identify an image region with a predominantly red wavelength spectrum, wherein the region of interest (20) is realigned in relation to or set as the image region with a predominantly red wavelength spectrum.

6. Method according to one of claims 1 to 5, wherein specular reflections are detected using high intensity information in the white light images, wherein areas comprising the specular reflections are deleted from the region of interest (20).

7. Method according to one of claims 1 to 6, wherein the image processing step of the method further comprises increasing a contrast between the fluorescence light emitted by the faint fluorescence source (16) and background.

8. Method according to claim 7, wherein the fluorescence images are captured using an image sensor at high gain settings and/or exposure.

9. Method according to claim 7 or 8, wherein the contrast between the fluorescence light (14) emitted by the faint fluorescence source (16) and background is increased by applying an inverse gamma correction.

10. Method according to one of claims 7 to 9, wherein the white light images and the fluorescence images are captured simultaneously or alternately.

11. Method according according to one of claims 7 to 10, wherein the image processing on the one or more fluorescence images comprises at least one of masking specular reflection, noise suppression and normalization.

12. Method according to claim 11, wherein the normalization comprises a percentile correction, in particular cutting off the darkest 1 to 2 % and the brightest 1 to 2 % of the pixels of the fluorescence image, and/or selecting a bottom part of the brightness spectrum and spreading the brightness information contained therein over the full brightness spectrum, wherein in particular the bottom part of the brightness spectrum includes 0% to 20%, in particular 0% to 10%, in particular 0% to 5%, of the brightness spectrum of the input fluorescence images.

13. System (1) for the visualization of faint fluorescence in surgery, in particular open surgery, comprising
- a control device (3) comprising an image processing unit (4),
- a light source device (5) configured to produce excitation light and white light,
- an image capturing device (8) configured to capture fluorescence images and white light images,
wherein the control device (3) is configured to control the operation of the light source device (5) and the image capturing device (8) and to carry out the method steps of the method according to one of claims 1 to 12.

14. Software program configured to execute the method steps of the method according to one of claims 1 to 12 when run on the control device (3) of the system (1) according to claim 13.
